# EUROPÄISCHE PATENTSCHRIFT

(11) **EP 0 830 117 B1**
(45) Veröffentlichungstag und Bekanntmachung des Hinweises auf die Patenterteilung: **08.03.2000**
(21) Anmeldenummer: 96916002.7
(22) Anmeldetag: 05.06.1996
(51) Int. Cl.: A61F 2/66, A61F 2/76

(54) **GELENKLOSES PROTHESENFUSSTEIL**
JOINTLESS FOOT PROSTHESIS
PROTHESE DE PIED DEPOURVUE D'ARTICULATIONS

(30) Priorität: 09.06.1995 DE 19521147
(43) Veröffentlichungstag der Anmeldung: 25.03.1998
(73) Patentinhaber: Otto Bock Orthopädische Industrie Besitz- und Verwaltungs-Kommanditgesellschaft, 37115 Duderstadt (DE)
(72) Erfinder: WELLERSHAUS, Ulf, D-37115 Duderstadt (DE); LEPPACK, Kai, D-37115 Duderstadt (DE)
(74) Vertreter: Gramm, Werner, Prof. Dipl.-Ing.
(86) Internationale Anmeldenummer: DE9601031
(87) Internationale Veröffentlichungsnummer: WO9641598

(56) Entgegenhaltungen:
- EP-A- 0 562 241
- WO-A-88/00815
- FR-A- 2 148 322
- FR-A- 2 410 998
- US-A- 4 387 472
- US-A- 4 555 817
- US-A- 4 645 509
- US-A- 5 112 356

## Beschreibung

Die Erfindung betrifft ein gelenkloses Prothesenfußteil mit einer sich vom Fersenbereich in den Vorfußbereich erstreckenden Fußinnenfeder, die von einer kosmetischen Verkleidung umschlossen und über einen Schraubbolzen mit einem Adapter verbunden ist, der eine Klemmverbindung zum lösbaren Anschluß eines Prothesenrohres aufweist.

Eine derartige Ausführungsform läßt sich beispielsweise der US-Patentschrift 4,645,509 entnehmen. Der Adapter ist bei dieser Ausführungsform durch den Schraubbolzen fest gegen eine obere Anschlagfläche der Fußinnenfeder geschraubt und ist dieser gegenüber unverschiebbar angeordnet. Der Adapter weist eine Klemmhülse auf, in die das untere Ende des Prothesenrohres der Unterschenkelprothese einschiebbar und durch Anziehen einer separaten Klemmschraube festklemmbar ist.

Die US 5,112,356 offenbart ein mit einem Kugelgelenk bestücktes Prothesenfußteil mit einer sich vom Fersenbereich in den Vorfußbereich erstreckenden Fußinnenfeder, die von einer kosmetischen Verkleidung umschlossen und über einen Schraubbolzen mit einem Adapter verbunden ist, der den unteren Teil des Kugelgelenkes bildet, dessen oberes Gelenkteil in das untere Ende einer geschlitzten Klemmhülse integriert ist, die zum lösbaren Anschluß eines Prothesenrohres dient. Der Adapter stützt sich mit seiner als Zylindersegment ausgeführten und mit einer Verzahnung versehenen Unterseite auf einer entsprechend ausgebildeten Anschlußfläche der Fußinnenfeder ab, deren Verzahnung formschlüssig in die Verzahnung des Adapters eingreift. Der Schraubbolzen ist durch einen sich in Fußlängsrichtung erstreckenden, eine dem Bolzendurchmesser entsprechende Breite aufweisenden Schlitz der Fußinnenfeder gesteckt und stützt sich mit seinem unteren Ende an der Unterseite eines oberen horizontalen Schenkels der Fußinnenfeder über ein sich über die Schlitzbreite erstreckendes Zylindersegment ab. Die Kugelgelenkverbindung läßt sich nur durch vollständiges Herausschrauben des Schraubbolzens aus dem Adapter trennen.

Die FR 2.148.322 offenbart einen gelenklosen Fuß, der keine Fußinnenfeder sondern einen Holzkern aufweist, auf dem ein Zylindersegment über Schrauben befestigt ist, dessen nach oben offene teilzylinderische Wandung mit einer Verzahnung versehen ist, auf der sich ein Adapter mit seiner als Zylindersegment ausgebildeten und mit einer Verzahnung versehenen Unterseite abstützt, wobei die beiden Verzahnungen formschlüsssig ineinandergreifen. Das nach oben ragende Ende des Adapters ist mit einer Ausnehmung versehen, in die das untere Ende eines Prothesenrohres gesteckt ist. Zur Bildung einer Klemmvorrichtung ist in das untere Ende des Prothesenrohres eine geschlitzte Hülse eingeschoben, die eine sich von oben nach unten konisch verjüngende Innenwandung aufweist, in der eine Spannmutter geführt ist, die über einen von unten durch den Holzkern und die beiden Zylindersegmente gesteckten Schraubbolzen höhenverstellbar ist. Durch Anziehen des Schraubbolzens wird die Spannmutter nach unten gezogen, spreizt dadurch die Spreizhülse auseinander und drückt so das Prothesenrohr bzw. die Wandung seines unteren Endes gegen die Innenwandung der Ausnehmung im Adapter.

Diese Klemmverbindung hat den Nachteil, daß die Klemmung eine Aufweitung des unteren Endes des Prothesenrohres erfordert, sodaß nur entsprechend dünnwandige Prothesenrohre oder aber nur geschlitzte Prothesenrohre Verwendung finden können. Vor allem aber führt das Dehnen des Rohres in dieser Region im Gebrauch des Prothesenfußteils zum Dauerbruch. Die Aufweitung des Prothesenrohres erfolgt auch nicht gleichmäßig über einen ausreichend langen Rohrabschnitt. Da die eigentliche Klemmverbindung zwischen Rohraußenwandung und Adapterinnenwandung erfolgen muß, der Adapter jedoch sehr starr ausgebildet sein muß, läßt sich eine tragene Klemmfläche kaum sicherstellen. Ein hoher Klemmdruck kann zum Aufreißen des Rohres bei Biegelast führen.

Der Erfindung liegt die Aufgabe zugrunde, das eingangs beschriebene Prothesenfußteil in seiner Funktion zu verbessern.

Diese Aufgabe wird gemäß der Erfindung in Verbindung mit den eingangs aufgeführten Merkmalen durch folgende zusätzliche Merkmale gelöst:
a) der Adapter stützt sich mit seiner als Zylindersegment ausgebildeten und mit einer Verzahnung versehenen Unterseite auf einer entsprechend ausgebildeten Anschlußfläche der Fußinnenfeder ab, deren Verzahnung formschlüssig in die Verzahnung des Adapters eingreift;
b) das obere Ende des Adapters ist als Zapfen ausgebildet, auf den das Prothesenrohr gesteckt ist;
c) der Schraubbolzen ist durch einen sich in Fußlängsrichtung erstreckenden, eine dem Bolzendurchmesser entsprechende Breite aufweisenden Schlitz der Fußinnenfeder gesteckt und stützt sich mit seinem unteren Ende an der Unterseite der Fußinnenfeder ab, die im Schlitzbereich ebenfalls als Zylindersegment ausgebildet ist;
d) das obere, sich über den Adapterzapfen hinaus erstreckende Ende des Schraubbolzens beaufschlagt die genannte Klemmverbindung, die ein in einem Ringspalt zwischen Adapterzapfen und Prothesenrohr angeordnetes Spreizelement aufweist, das durch Anziehen des Schraubbolzens beaufschlagt wird.
e) das Spreizelement weist zumindest einen Elastomerring (14, 16) auf, der sich mit seiner Unterseite direkt oder indirekt auf einer Anschlagfläche (10) des Adapters (5) abstützt und auf seiner Oberseite von einem auf dem Adapterzapfen (9) geführten Druckstück (18) beaufschlagt wird, das seinerseits in Axialrichtung des Schraubbolzens (6) von diesem beaufschlagt wird.

Dieses erfindungsgemäße Prothesenfußteil ermöglicht über die vorgesehene Rasteneinstellung die Einstellung verschiedener Absatzhöhen. Zentrierung und Klemmung des Prothesenrohres erfolgen durch das genannte Spreizelement.

Zur Verbesserung der Zentrierung des Prothesenrohres, zum Schutz des Elastomerringes sowie für einen etwaigen Längenausgleich gegenüber dem Druckstück ist der Elastomerring zwischen einem Zwischen- und einem Abschlußring angeordnet.

Zur Aufrechterhaltung einer bestimmten, in Bolzenlängsrichtung verlaufenden Zugkraft ist es vorteilhaft, wenn sich der Schraubbolzen über ein Federelement auf dem Druckstück abstützt.

Weitere Merkmale der Erfindung sind Gegenstand der Unteransprüche und werden mit weiteren Vorteilen der Erfindung anhand eines Ausführungsbeispieles näher erläutert.

In der Zeichnung ist eine als Beispiel dienende Ausführungsform der Erfindung dargestellt. Es zeigen:

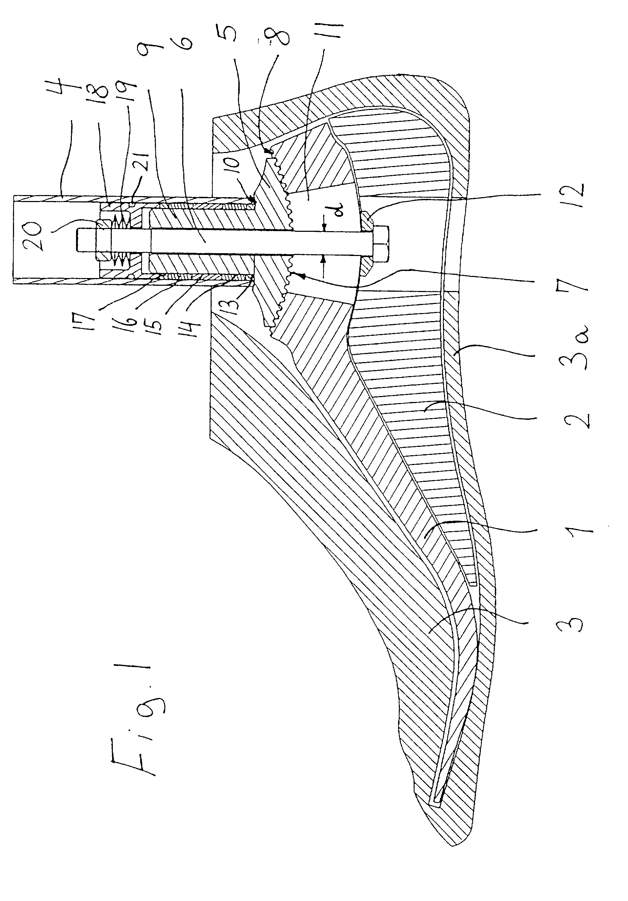

- Figur 1: einen lotrechten Längsschnitt durch ein Prothesenfußteil mit angesetztem Prothesenrohr und

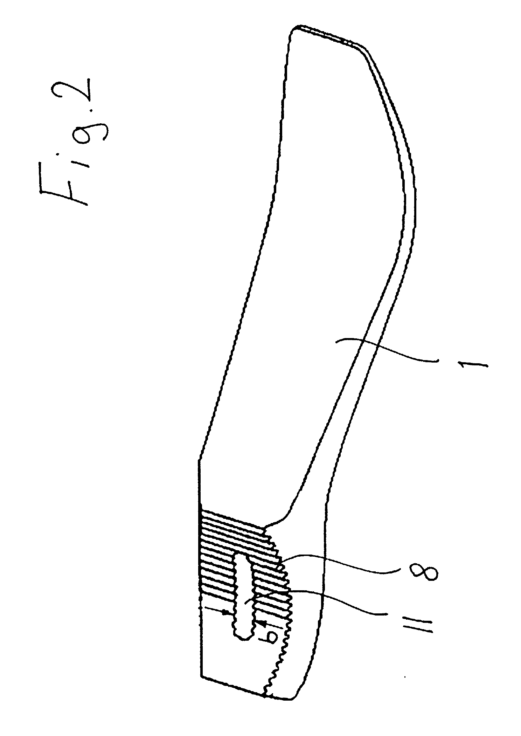

- Figur 2: in schaubildlicher Darstellung die Fußinnenfeder gemäß Figur 1 jedoch in gegenüber der Figur 1 um 180° verdrehter Lage.

Das dargestellte Prothesenfußteil umfaßt im wesentlichen eine sich vom Fersenbereich in den Vorfußbereich erstreckende Fußinnenfeder 1, die sich auf einem auswechselbaren Einlegekeil 2 abstützt, der formschlüssig zwischen der Fußinnenfeder 1 und der Unterseite 3a einer Fußinnenfeder 1 und Einlegekeil 2 umschließenden kosmetischen Verkleidung 3 gehalten ist. Letztere kann als Schaumkosmetik, als Schaumkosmetik mit verstärkter Sohle oder als Schuh bzw. Sandale ausgebildet sein.

Die Verbindung des Prothesenfußteils mit einem Prothesenrohr 4 einer Unterschenkelprothese erfolgt über einen Adapter 5 und einen sich durch diesen hindurch erstreckenden Schraubbolzen 6.

Der Adapter 5 stützt sich mit seiner als Zylindersegment ausgebildeten und mit einer Verzahnung 7 versehenen Unterseite auf einer entsprechend ausgebildeten Anschlußfläche der Fußinnenfeder 1 ab, deren Verzahnung 8 formschlüssig in die Verzahnung 7 des Adapters 5 eingreift. Das obere Ende des Adapters 5 ist als Zapfen 9 ausgebildet, auf den das Prothesenrohr 4 gesteckt ist, das sich mit seinem unteren Ende auf einer ringförmigen Anschlagfläche 10 des Adapters 5 abstützt. Der Schraubbolzen 6 ist durch einen sich in Fußlängsrichtung erstreckenden, eine dem Bolzendurchmesser d entsprechende Breite b aufweisenden Schlitz 11 der Fußinnenfeder 1 gesteckt und stützt sich mit seinem das untere Ende bildenden Bolzenkopf über eine Scheibe 12 an der Unterseite 1a der Fußinnenfeder 1 ab, die in dem den Schlitz 11 umgebenden Bereich ebenfalls als Zylindersegment ausgebildet ist, deren Kontur die Auflagefläche der Scheibe 12 angepaßt ist.

Das Prothesenrohr 4 umschließt zusammen mit dem Zapfen 9 des Adapters 5 einen Ringspalt, der - gesehen von der Adapter-Anschlagfläche 10 nach oben - einen unteren, vorzugsweise aus Kunststoff bestehenden Abschlußring 13, einen Elastomerring 14, einen Kunststoff-Zwischenring 15, einen weiteren Elastomerring 16 sowie einen oberen Kunststoff-Abschlußring 17 aufnimmt. Letzterer wird von oben beaufschlagt von einem auf dem Adapterzapfen 9 geführten, sich an der Innenwandung des aufgesteckten Prothesenrohres 4 abstützenden Druckstück 18. Der Schraubbolzen 6 ist mit seinem oberen Ende durch das Druckstück 18 sowie auf diesem aufliegende Tellerfedern 19 hindurchgesteckt und mit einer Befestigungsmutter 20 bestückt, die für die Tellerfedern 19 das zweite, dem Druckstück 18 gegenüberliegende Widerlager bildet. Zwischen dem Druckstück 18 und der Befestigungsmutter 20 ist eine in der Zeichnung nicht näher dargestellte Verdrehsicherung vorgesehen. Ferner ist zwischen dem Druckstück 18 und dem Prothesenrohr 4 eine Verdrehsicherung vorgesehen in Form eines dazwischen eingelegten Elastomerringes 21.

Nach dem (teilweise) Lösen des Schraubbolzens 6 besteht die Möglichkeit, verschiedene Absatzhöhen durch Verschwenken des Adapters 5 gegenüber der Fußinnenfeder 1 innerhalb der durch den Schlitz 11 definierten lotrechten Ebene einzustellen. In der gewünschten verschwenkten Stellung wird dann der Schraubbolzen 6 wieder angezogen, so daß die gewünschte Winkelstellung über die ineinandergreifenden Verzahnungen 7, 8 formschlüssig gesichert ist. Die für die Einstellung vorgenommene Verschwenkung des Schraubbolzens 6 und damit des Adapters 5 erfolgt um eine gedachte horizontale Schwenkachse, die bei dem Ausführungsbeispiel etwa im Bereich der Befestigungsmutter 20 liegt und senkrecht auf der Zeichenebene steht.

Der Schraubbolzen 6 verbindet als zentrale Halterung alle Prothesenfußteile miteinander und sichert deren Position in bezug auf deren Verschiebbarkeit in Richtung der Beinlängsachse. Gleichzeitig dient der Schraubbolzen 6 aber auch zum Aufbringen der das aufgesteckte Prothesenrohr 4 kraftschlüssig mit dem Adapterzapfen 9 verbindenden Kraft. Denn ein Anziehen des Schraubbolzens 6 beaufschlagt das Druckstück 18 mit einer in Bolzenlängsrichtung nach unten weisenden Kraft, durch die das im Ringspalt zwischen Prothesenrohr 4 und Adapterzapfen 9 angeordnete Ringpaket axial beaufschlagt wird. Diese Kraftbeaufschlagung führt zu einer Verformung der Elastomerringe 14, 16, die letztlich den genannten Kraftschluß hervorrufen. Hierdurch wird das Prothesenrohr 4 gegenüber dem Adapterzapfen 9 hinsichtlich der Längs- und Torsionskräfte gesichert. Zugleich aber ergibt sich eine schnell und einfach lösbare Verbindung zwischen dem Prothesenfußteil und dem Prothesenrohr 4. Das durch die Tellerfedern 19 gebildete Federelement steuert - ohne Verwendung eines Drehmomentenschlüssels - das notwendigerweise aufzubringende Drehmoment zum Anziehen der Schraube.

Vom Aufbau her kann das Prothesenfußteil links- und rechtsseitig verwendet werden.

Die Fußinnenfeder 1 dient zur Energiespeicherung beim Abrollen und der dadurch bewirkten Biegung an dem sich verjüngenden Ende gegenüber dem Adapter 5. Ein seitliches Verrutschen des Adapters 5 wird durch die Formgebung des Schlitzes 11 verhindert.

Der Einlegekeil 2 dient zum Anpassen des Fußteils an die Absatzhöhe; durch entsprechende Wahl des Einlegekeils läßt sich die Auftrittsdämpfung verändern. Die kosmetische Verkleidung 3 erlaubt eine einfache Anpassung an die Fußgröße.

Das Prothesenrohr 4 dient zur Überleitung von Kräften entlang der Beinlängsachse auf das Prothesenfußteil; durch Kürzen des Prothesenrohres 4 ist in einfacher Weise eine individuelle Einstellung der Beinlänge möglich.

## Patentansprüche

1. Gelenkloses Prothesenfußteil mit einer sich vom Fersenbereich in den Vorfußbereich erstreckenden Fußinnenfeder (1), die von einer kosmetischen Verkleidung (3) umschlossen und über einen Schraubbolzen (6) mit einem Adapter (5) verbunden ist, der eine Klemmverbindung zum lösbaren Anschluß eines Prothesenrohres (4) aufweist, mit folgenden Merkmalen:
a) der Adapter (5) stützt sich mit seiner als Zylindersegment ausgebildeten und mit einer Verzahnung (7) versehenen Unterseite auf einer entsprechend ausgebildeten Anschlußfläche der Fußinnenfeder (1) ab, deren Verzahnung (8) formschlüssig in die Verzahnung (7) des Adapters (5) eingreift;
b) das obere Ende des Adapters (5) ist als Zapfen (9) ausgebildet, auf den das Prothesenrohr (4) gesteckt ist;
c) der Schraubbolzen (6) ist durch einen sich in Fußlängsrichtung erstreckenden, eine dem Bolzendurchmesser (d) entsprechende Breite (b) aufweisenden Schlitz (11) der Fußinnenfeder (1) gesteckt und stützt sich mit seinem unteren Ende an der Unterseite (1a) der Fußinnenfeder (1) ab, die im Schlitzbereich ebenfalls als Zylindersegment ausgebildet ist;
d) das obere, sich über den Adapterzapfen (9) hinaus erstreckende Ende des Schraubbolzens (6) beaufschlagt die genannte Klemmverbindung, die ein in einem Ringspalt zwischen Adapterzapfen (9) und Prothesenrohr (4) angeordnetes Spreizelement (14, 16) aufweist, das durch Anziehen des Schraubbolzens (6) beaufschlagt wird;
e) das Spreizelement weist zumindest einen Elastomerring (14, 16) auf, der sich mit seiner Unterseite direkt oder indirekt auf einer Anschlagfläche (10) des Adapters (5) abstützt und auf seiner Oberseite von einem auf dem Adapterzapfen (9) geführten Druckstück (18) beaufschlagt wird, das seinerseits in Axialrichtung des Schraubbolzens (6) von diesem beaufschlagt wird.

2. Prothesenfußteil nach Anspruch 1, **dadurch gekennzeichnet,** daß der Elastomerring (14, 16) zwischen einem Zwischen- und einem Abschlußring (15, 13, 17) angeordnet ist.

3. Prothesenfußteil nach Anspruch 1 oder 2, **dadurch gekennzeichnet**, daß sich der Schraubbolzen (6) über ein Federelement (19) auf dem Druckstück (18) abstützt.

4. Prothesenfußteil nach einem der vorhergehenden Ansprüche, **dadurch gekennzeichnet,** daß sich die Fußinnenfeder (1) auf einem auswechselbaren Einlegekeil (2) abstützt.

5. Prothesenfußteil nach Anspruch 4, **dadurch gekennzeichnet,** daß der Einlegekeil (2) formschlüssig zwischen der Unterseite (3a) der kosmetischen Verkleidung (3) und der Fußinnenfeder (1) gehalten ist.

## Claims

1. Joint-less prosthetic foot part with a foot internal tongue (1) which extends from the heel region into the metatarsal region, is surrounded by a cosmetic cladding (3) and is connected via a screw bolt (6) to an adapter (5) which has a clamping connection for the detachable connection of a prosthetic tube (4), comprising the following features:
a) with its underside provided with a toothing (7) and formed as a cylinder segment, the adapter (5) is supported on a correspondingly formed connecting surface of the foot internal tongue (1), the toothing (8) of which positively engages into the toothing (7) of the adapter (5);
b) the upper end of the adapter (5) is formed as peg (9) onto which the prosthetic tube (4) is placed;
c) the screw bolt (6) is placed through a slit (11) of the foot internal tongue (1) extending in the longitudinal direction of the foot and having a width (b) corresponding to the bolt diameter (d) and with its lower end is supported on the underside (la) of the foot internal tongue (1) which is formed as a cylinder segment in the slit region also;
d) the upper end of the screw bolt (6) extending beyond the adapter peg (9) acts upon the said clamping connection, which has a splaying element (14, 16) which is arranged in an annular gap between adapter peg (9) and prosthetic tube (4) and is acted upon by tightening the screw bolt (6) ;
e) the splaying element has at least one elastomer ring (14, 16) which is supported with its underside directly or indirectly on a stop surface (10) of the adapter (5) and is acted upon on its upper side by a pressure piece (18) guided on the adapter peg (9), which pressure piece is acted upon in its turn by the screw bolt in the axial direction of the screw bolt (6).

2. Prosthetic foot part according to Claim 1, characterized in that the elastomer ring (14, 16) is arranged between an intermediate and a terminal ring (15, 13, 17).

3. Prosthetic foot part according to Claim 1 or 2, characterized in that the screw bolt (6) is supported on the pressure piece (18) by means of a spring element (19).

4. Prosthetic foot part according to one of the preceding Claims, characterized in that the foot internal tongue (1) is supported on an interchangeable insert wedge (2) .

5. Prosthetic foot part according to Claim 4, characterized in that the insert wedge (2) is positively held between the underside (3a) of the cosmetic cladding (3) and the foot internal tongue (1) .

## Revendications

1. Prothèse du pied sans articulation, comportant un ressort intérieur du pied (1) qui s'étend de la zone du talon dans la zone de la pointe du pied, est enfermé dans un habillage cosmétique (3) et est relié par une vis (6) avec un adaptateur (5) qui présente une liaison par serrage pour le raccordement démontable d'un tube de prothèse (4), comprenant les particularités suivantes :
a) par son côté inférieur réalisé sous la forme d'un segment cylindrique et muni d'une denture (7) l'adaptateur (5) s'appuie sur une surface de raccordement du ressort intérieur du pied (1), formée de façon correspondante, dont la denture (8) s'engage avec verrouillage par forme conjuguées dans la denture (7) de l'adaptateur (5);
b) l'extrémité supérieure de l'adaptateur (5) est réalisée sous la forme d'un embout (9) sur lequel est enfilé le tube de prothèse (4);
c) la vis (6) est enfilée à travers une fente (11) du ressort intérieur du pied (1), cette fente présentant une largeur (b) correspondant au diamètre (d) de la vis et s'étendant selon la direction longitudinale du pied, la vis s'appuyant par son extrémité inférieure sur le côté inférieur (1a) du ressort intérieur du pied (1), qui dans la région de la fente est également réalisé sous la forme d'un segment cylindrique;
d) l'extrémité supérieure de la vis (6) qui dépasse de l'embout (9) de l'adaptateur sollicite la liaison par serrage précitée, qui présente un élément d'expansion (14, 16) disposé entre l'embout (9) de l'adaptateur et le tube de prothèse (4), cet élément d'expansion étant sollicité par serrage de la vis (6);
e) l'élément d'expansion présente au moins une bague élastomère (14, 16) qui par son côté inférieur s'appuie directement ou indirectement sur une surface de butée (10) de l'adaptateur (5) et se trouve sollicité sur son côté supérieur par une pièce presseuse (18) guidée sur l'embout (9) de l'adaptateur, elle-même sollicitée par la vis (6) en direction axiale de cette dernière.

2. Prothèse du pied selon la revendication 1, caractérisée en ce que la bague élastomère (14, 16) est disposée entre une bague intermédiaire et une bague terminale (15, 13, 17).

3. Prothèse du pied selon la revendication 1 ou 2, caractérisée en ce que la vis (6) s'appuie sur la pièce presseuse (18) par l'intermédiaire d'un élément élastique (19).

4. Prothèse du pied selon l'une des revendications précédentes, caractérisée en ce que le ressort intérieur du pied (1) s'appuie sur une cale d'insertion (2) interchangeable.

5. Prothèse du pied selon la revendication 4, caractérisée en ce que la cale d'insertion (2) est maintenue par formes conjuguées entre le côté inférieur (3a) de l'habillage cosmétique (3) et le ressort intérieur du pied (1).
